(19) Europäisches Patentamt
European Patent Office
Office européen des brevets

(11) **EP 4 285 821 A1**

(12) **EUROPEAN PATENT APPLICATION**

(43) Date of publication:
**06.12.2023 Bulletin 2023/49**

(21) Application number: **22172894.2**

(22) Date of filing: **12.05.2022**

(51) International Patent Classification (IPC):
**A61B 5/242** (2021.01)    **A61B 5/311** (2021.01)
**A61B 5/388** (2021.01)

(52) Cooperative Patent Classification (CPC):
**A61B 5/311; A61B 5/242; A61B 5/388; A61B 5/40;**
**A61B 5/6852;** A61B 5/05; A61B 5/201;
A61B 2560/0209; A61B 2560/0223;
A61B 2562/0223

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **KAHLERT, Joachim Johannes**
**Eindhoven (NL)**

• **DOODEMAN, Gerardus Johannes Nicolaas**
**Eindhoven (NL)**
• **SIO, Charles Frederik**
**Eindhoven (NL)**
• **WORTELBOER, Pippinus Maarten Robertus**
**Eindhoven (NL)**
• **FRACKOWIAK, Bruno Jean François**
**Eindhoven (NL)**

(74) Representative: **Philips Intellectual Property &**
**Standards**
**High Tech Campus 52**
**5656 AG Eindhoven (NL)**

(54) **DETECTING NERVE ACTIVITY**

(57)    An interventional device (110) for detecting nerve activity, is provided. The interventional device includes: a magnetic sensor (120) that is coupled to an insertable portion of the interventional device. The magnetic sensor (120) is configured to generate signals in response to magnetic fields produced by nerve activity.

A system (200) is also provided. The system includes the interventional device (110) and a controller (140) that is configured to receive the signals generated by the magnetic sensor (120), and to output a detection result indicative of nerve activity in response to the received signals.

FIG. 11

EP 4 285 821 A1

## Description

## Technical Field

The present disclosure relates to the detection of nerve activity. An interventional device, a system, a computer-implemented method, and a computer program product, are disclosed.

## Background

An assessment of nerve activity is performed in various medical procedures in order to investigate neurological behaviour. For example, brain function is monitored via electroencephalography "EEG". Nerve activity may also be monitored during a renal denervation procedure. Renal denervation is an interventional procedure to treat drug-resistant hypertension, i.e. elevated blood pressure. Renal denervation is typically performed by ablating the (sympathetic) nerves between the central nervous system and the kidneys, usually in the vicinity of the renal arteries. This procedure is typically performed using an interventional device such as an ablation catheter. The effect of the ablation procedure is to remove the stimulant for the elevated blood pressure. However, the relatively low clinical success rate of renal denervation, as measured via a long-term reduction in blood pressure combined with the high cost of the procedure, present barriers to its wider adoption. Quantification of nerve activity may help to increase the clinical success rate of renal denervation. Procedure improvements may involve a more accurate localisation of the nerves, a better stratification of patients for a denervation therapy, and a more direct and comprehensive validation of the procedure's success.

When a nerve is activated, it triggers an electrical impulse, often referred to as an "action potential" that propagates in a forward direction along the associated nerve fiber, thus seemingly transmitting the pulse. The action potential is difficult to quantify without puncturing tissue in order to bring an electrode in very close vicinity or in contact with the nerve fiber. Such drawbacks hamper the electrical measurement of nerve activity. However, the time varying electrical impulse in a nerve fiber generates a magnetic field around the nerve fiber. This magnetic field is in the sub-picotesla range, and permits the measurement of nerve activity using a magnetic sensor.

Various magnetic sensors have been used to measure the activity of nerve cells, i.e. neurons. For instance, in a Magnetoencephalography "MEG" system, superficial sensors are positioned around the head. Magnetic sensors such as inductive sensors, superconducting quantum interference device "SQUID" sensors, and optically pumped magnetometer "OPM" sensors, have been used to sense the magnetic field generated by the activity of the neurons in the brain. The strength of this magnetic field decreases rapidly with distance from the neurons, or nerve. The cumulative field generated by large numbers of neurons can however be detected by a magnetic sensor located on the skin, i.e. a few centimetres from the source. Similar sensors have been used in Magnetocardiography "MCG" systems in order to monitor the magnetic field emitted by the beating heart from a superficial location. In an MCG system, the magnetic sensors are located on the chest and close to the heart. In this setting, many millions of nerves and muscle fibers are active simultaneously, generating a cumulative signal that is sufficiently strong to be detected by a sensor placed on the skin, several centimetres away from the nerves.

The magnetic fields that are emitted by some nerves can, however, be much smaller. This is the case for renal nerves, which contain only hundreds to a few thousands of nerve fibers. The renal nerves are located in the tunica adventitia (outer layer) of the renal artery wall, and extend alongside the renal arteries. The magnetic fields generated by renal nerves decay rapidly with distance, and consequently they are too small to be sensed by superficial sensors located on the skin. Thus, in order to detect the magnetic fields generated by some types of nerves such as renal nerves, it becomes necessary to insert a magnetic sensor into the body and to position the magnetic sensor close to the nerve(s) of interest. This, in turn places constraints on the size of magnetic sensors that may be used to detect nerve activity in an interventional setting. Also, the magnitude of the magnetic field close to nerves varies strongly with the sensing location. Moreover, it can be challenging to obtain an accurate measurement of the magnetic fields from nerves in the presence of magnetic interference from other sources.

WO2013/096461 A1 describes an apparatus for locally monitoring nerve activity that may be incorporated into a nerve ablation catheter. The catheter is equipped with magnetic sensing for both identifying nerves and assessing the success of the ablation. The catheter is also equipped with an ablation instrument for both stimulating and destroying nerve tissue. In the disclosed apparatus, the magnetic sensor is configured to sense magnetic fields that are generated in response to an artificial activation of the nerve by the ablation instrument.

However, there remains a need to improve the detection of induced and/or natural nerve activity in an interventional device.

## Summary

According to one aspect of the present disclosure, an interventional device for detecting nerve activity, is provided. The interventional device includes a magnetic sensor. The magnetic sensor is coupled to an insertable portion of the interventional device. The magnetic sensor is configured to generate signals in response to magnetic fields produced by nerve activity.

According to another aspect of the present disclosure, a system is provided. The system includes the interventional device, and a controller. The controller is configured to receive the signals generated by the magnetic sensor, and to output a detection result indicative of nerve activity in response to the received signals.

Further aspects, features, and advantages of the present disclosure will become apparent from the following description of examples, which is made with reference to the accompanying drawings.

## Brief Description of the Drawings

Fig. 1 is a schematic diagram illustrating an example of a cross section of a vessel VES shrouded by bundles of nerve fibers $BNF_{1..3}$, and an interventional device ID, in accordance with some aspects of the present disclosure.

Fig. 2 is a schematic diagram illustrating an example of a typical action potential signal on a nerve fiber, in accordance with some aspects of the present disclosure.

Fig. 3 is a schematic diagram illustrating a) an example of a model of a nerve fiber extending along and adjacent to a vessel lumen, and b) modelled electric dipole currents representing the depolarization and repolarization of the nerve fiber, in accordance with some aspects of the present disclosure.

Fig. 4 is a schematic diagram illustrating an example of a) the simulated magnetic flux density and b) corresponding streamlines, around a nerve fiber, in accordance with some aspects of the present disclosure.

Fig. 5 is a diagram illustrating an example of a) a model of a dipole current in a nerve fiber with a sensor located at radial distance $r$, b) a predicted magnetic flux density arising from the modelled dipole current, c) the predicted magnetic flux density at various radial distances, $r$, and d) the distribution of the arrival times of the magnetic flux density used to generate the predicted magnetic flux density in c), in accordance with some aspects of the present disclosure.

Fig. 6 is a schematic diagram illustrating an example of an interventional device 110 for detecting nerve activity, in accordance with some aspects of the present disclosure.

Fig. 7 is a schematic diagram illustrating a first example of a magnetic sensor 120 that includes an optically pumped magnetometer, in accordance with some aspects of the present disclosure.

Fig. 8 is a schematic diagram illustrating a second example of a magnetic sensor 120 that includes an optically pumped magnetometer, in accordance with some aspects of the present disclosure.

Fig. 9 is a schematic diagram illustrating a third example of a magnetic sensor 120 that includes an optically pumped magnetometer, in accordance with some aspects of the present disclosure.

Fig. 10 is a schematic diagram illustrating a fourth example of a magnetic sensor 120 that includes an optically pumped magnetometer, in accordance with some aspects of the present disclosure.

Fig. 11 is a schematic diagram illustrating an example of a system 200 that includes an interventional device 110 for detecting nerve activity, and a controller 140, in accordance with some aspects of the present disclosure.

Fig. 12 is a schematic diagram illustrating examples of a) a magnitude of a power $P_{HEATER}$ applied to a heater of an optical cell of an OPM, b) a resulting temperature $T_{CELL}$ of the optical cell, c) a magnetic field $B_{NERVE}$ generated by an activated nerve, and d) a signal $B_{DETECTED}$ generated by the OPM in response to a detection of the magnetic field $B_{NERVE}$, in accordance with some aspects of the present disclosure.

Fig. 13 is a schematic diagram illustrating an example of a cross section of a vessel VES shrouded by bundles of nerve fibers $BNF_{1..3}$, and an interventional device ID including an expandable balloon, in accordance with some aspects of the present disclosure.

Fig. 14 is a graph illustrating a) a signature of a pulse train representing the magnetic field detected from a renal nerve in a non-hypertensive subject and b) a signature of a pulse train representing the magnetic field detected from a renal nerve in a hypertensive subject in whom the renal nerve activity is increased, in accordance with some aspects of the present disclosure.

Fig. 15 illustrates a plot of the pulse repetition time within a pulse train, Rt, against the number of pulses in a pulse train, nP, for simulated magnetic fields in a group of non-hypertensive subjects (circle symbols) and hypertensive subjects (stars), in accordance with some aspects of the present disclosure.

Fig. 16 illustrates an example of a system 200 that includes nested magnetic field shielding arrangement, in accordance with some aspects of the present disclosure.

Fig. 17 illustrates an example of a system 200 that includes an interventional device 110, a motion sensor 160, and one or more coils 150, SSL that are configured to generate a magnetic field for compensating for a background magnetic field detected by the magnetic sensor 120, in accordance with some aspects of the present disclosure.

## Detailed Description

Examples of the present disclosure are provided with reference to the following description and figures. In this description, for the purposes of explanation, numerous specific details of certain examples are set forth.

Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity. For instance, features described in relation to an interventional device, may be implemented in a system, and also in a computer-implemented method and a computer program product, in a corresponding manner.

In the following description, reference is made to an interventional device that includes a magnetic sensor. The magnetic sensor is configured to generate signals in response to magnetic fields produced by nerve activity. In some examples, reference is made an interventional device in the form of an ablation device. In some examples, the ablation device is an ablation catheter. However, it is to be appreciated that the interventional device may alternatively be a different type of device to an (RF/ High Frequency Ultrasound "HIFU"/ microwave) ablation device. For example, the interventional device may alternatively be a (guide) catheter, an (RF/ High Frequency Ultrasound "HIFU"/ microwave) ablation catheter, or a guidewire, or an intravascular ultrasound "IVUS" device, or an Optical Coherence Tomography "OCT" device, or a blood pressure sensing device and/or a blood flow sensing device, or a TEE probe, and so forth. Thus, the interventional device may in general be any type of interventional device.

Reference is also made herein to examples in which the interventional device, in the form of an ablation device, is used in a renal denervation procedure. The example ablation device is provided in the form of an ablation catheter that is adapted for insertion into the renal artery. However, it is to be appreciated that this serves only as an example, and that the interventional device may be adapted for insertion into the body in general. The interventional device may be adapted for insertion into various tissues in the body, or it may be adapted for insertion into various lumens in the body. In some examples, the interventional device is adapted for intravascular insertion, i.e. insertion into a vein or an artery. It is also contemplated that the interventional device may alternatively be adapted for insertion into other lumens in the body, including for example the digestive tract, the colon, the esophagus, the lungs, urinary tract, nasal cavity, and so forth. It is also contemplated that in some applications the interventional device may be used in open surgery. Moreover, it is to be appreciated that the interventional device may be used in clinical procedures in general, and that the device is not limited to use in the example renal denervation procedure described herein. The interventional device may for instance be a sensing device with which magnetic fields are sensed and no treatment is performed at all.

Reference is also made herein to operations that are performed by a controller. As described in more detail below, the controller may include one or more processors for performing some of these operations. It is noted that the operations that are performed by the controller may be provided in the form of computer-implemented methods. The computer implemented methods may be provided as a non-transitory computer-readable storage medium including computer-readable instructions stored thereon, which, when executed by at least one processor, cause the at least one processor to perform the method. In other words, the computer-implemented methods may be implemented in a computer program product. The computer program product can be provided by dedicated hardware, or hardware capable of running the software in association with appropriate software. When provided by a processor, or controller, the functions of the method features can be provided by a single dedicated processor, or by a single shared processor, or by a plurality of individual processors, some of which can be shared. The functions of one or more of the method features may for instance be provided by processors that are shared within a networked processing architecture such as a client/server architecture, a peer-to-peer architecture, the Internet, or the Cloud.

The explicit use of the terms "processor" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Furthermore, examples of the present disclosure can take the form of a computer program product accessible from a computer-usable storage medium, or a computer-readable storage medium, the computer program product providing program code for use by or in connection with a computer or any instruction execution system. For the purposes of this description, a computer-usable storage medium or a computer readable storage medium can be any apparatus that can comprise, store, communicate, propagate, or transport a program for use by or in connection with an instruction execution system, apparatus, or device. The medium can be an electronic, magnetic, optical, electromagnetic, infrared, or a semiconductor system or device or propagation medium. Examples of computer-readable media include semiconductor or solid state memories, magnetic tape, removable computer disks, random access memory "RAM", read-only memory "ROM", rigid magnetic disks and optical disks. Current examples of optical disks include compact disk-read only memory "CD-ROM", compact disk-read/write "CD-R/W", Blu-Ray™ and DVD.

As mentioned above, there remains a need to improve the detection of induced and/or natural nerve activity in an interventional device.

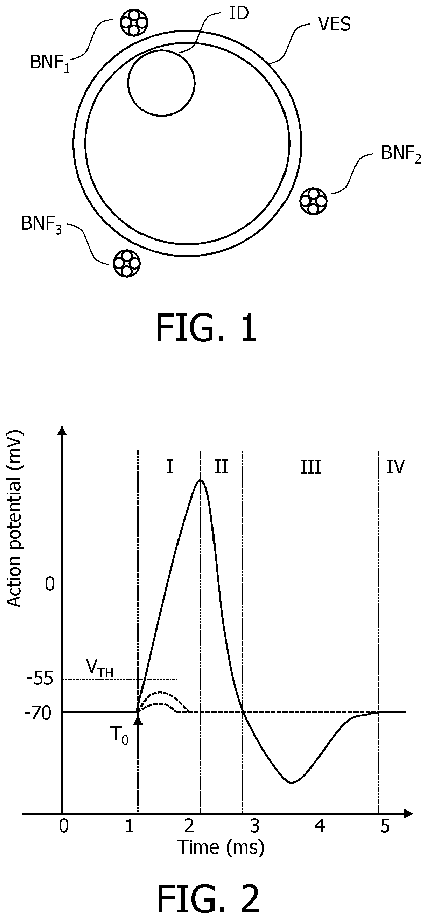

Fig. 1 is a schematic diagram illustrating an example of a cross section of a vessel VES shrouded by bundles of nerve fibers $BNF_{1..3}$, and an interventional device ID, in accordance with some aspects of the present disclosure. By way of an example, the vessel VES illustrated in Fig. 1 may represent the renal artery in a subject, and the bundles of nerve fibers $BNF_{1..3}$ may represent renal nerves. It may be desired to measure an activity of the renal nerves represented by the bundles of nerve fibers $BNF_{1..3}$ during a clinical investigation. For example, it may be desired to measure the activity of the renal nerves in order to stratify patients for treatment by a given procedure. Stratification of patients may comprise criteria based on which eligibility of a patient to a successful renal denervation treatment can be predicted or projected. The interventional device ID may for example be a sensing catheter that is inserted into the body from the groin area and navigated to the renal artery, VES, via the vasculature. The interventional device ID may be advanced through the vasculature to the renal artery through a guide catheter. Having reached the renal artery, VES, a section of the interventional device, ID, may be positioned close to the wall of the vessel VES in order to measure the magnetic fields emitted by the renal nerves represented by one of one of the bundles of nerve fibers $BNF_{1..3}$. By way of another example, it may be useful to measure the magnetic fields emitted by the renal nerves represented by one of one of the bundles of nerve fibers $BNF_{1..3}$ during a treatment procedure on the renal nerves. In this example, the interventional device ID may be an ablation catheter. With continued reference to Fig. 1, the treatment of renal nerves may involve the application of energy from the ablation catheter to one of the bundles of nerve fibers $BNF_{1..3}$ in order to suppress their activation, and to thereby treat hypertension in the subject. The ablation catheter may treat the bundle of nerve fibers BNFi by applying thermal, high frequency ultrasound "HIFU", microwave, radiofrequency "RF", or another form of ablation energy to the bundle of nerve fibers BNFi through the wall of the vessel VES. During the ablation treatment procedure, it may be useful to measure the activity of the treated bundle of nerve fibers BNFi in order to determine the effectiveness of the treatment.

As mentioned above, when a nerve is activated, it transmits an electrical impulse, often referred-to as an "action potential" along the associated nerve fiber. Fig. 2 is a schematic diagram illustrating an example of a typical action potential signal on a nerve fiber, in accordance with some aspects of the present disclosure. The electric action potential illustrated in Fig. 2 may be generated by one of the bundles of nerve fibres $BNF_{1..3}$ illustrated in Fig. 1, for example. The action potential illustrated in Fig. 2 represents the electrical potential at a point on the nerve fiber as the electrical impulse travels past. Prior to any activation, the potential on the nerve fiber is at a resting potential of approximately -70mV. A stimulation is applied at time To. If the stimulation is sufficiently large, i.e. it is greater than a threshold potential $V_{TH}$ of approximately -55mV, depolarization occurs during period I in Fig. 2, followed by repolarization during period II. After refractory period III, the potential on the nerve fiber returns to the resting potential of approximately -70mV, during period IV.

The action potential signal illustrated in Fig. 2 can be seen as a wave that is generated by successive depolarization and repolarization across the nerve cell membrane (axon), and is caused by the motion of its ions across its successive ion channels. No net current flows along the nerve fiber, as it would be expected in an electrical wire. The propagating wave nature of the action potential signal is due to a small delay between depolarization and repolarization, and the inability to reactivate immediately the previous channel as it is not yet repolarized . The ion motion (or toggling) in/out across one channel of the cell membrane triggers the next channel after a certain delay, and so on. From a fixed point on the nerve, the electrical impulse appears as a time-varying action potential.

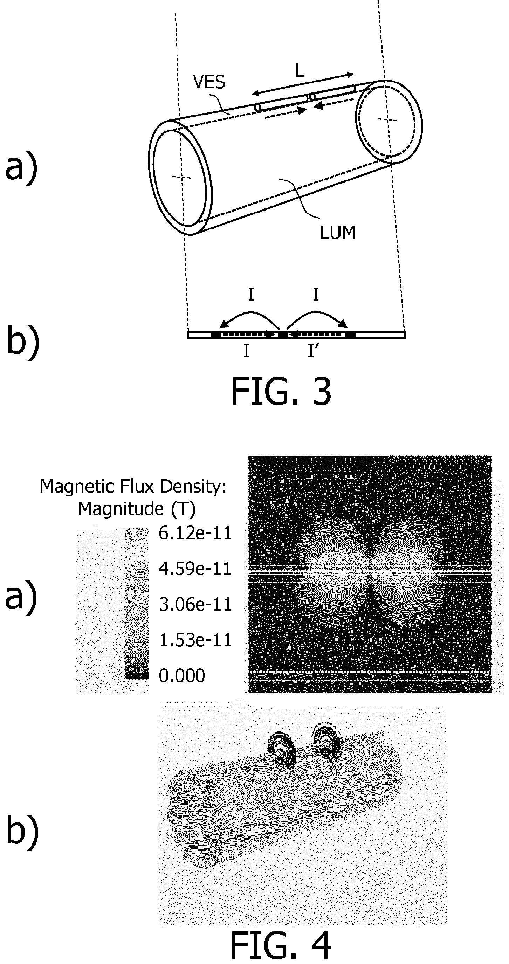

The typical action potential signal illustrated in Fig. 2 was used to generate a model, with which to simulate a magnetic field that may be measured in the vicinity of a nerve fiber. Various aspects of this model are illustrated in Fig. 3, which is a schematic diagram illustrating a) an example of a model of a nerve fiber extending along and adjacent to a vessel lumen, and b) modelled electric dipole currents representing the depolarization and repolarization of the nerve fiber, in accordance with some aspects of the present disclosure. In general, the wavelike propagation of the action potential along a nerve fiber is difficult to capture in a biophysical model because it involves charges (ions) toggling in multiple channels located all along the nerve at different times. This makes the problem unstable along the relatively large spatial distances along the nerve fiber. Thus, in the model illustrated in Fig. 3, an estimate of the magnetic field distribution around a renal nerve was modelled by approximating the action potential with electrical currents flowing in opposing electrical dipoles which are aligned with the direction of the nerve fiber, as illustrated in Fig. 3a). The electrical dipoles illustrated in Fig. 3a) have a combined length L. The resulting electrical currents, I and I', are illustrated in Fig. 3b). This approximation converts the unstable problem into a steady state model, and wherein the opposing dipoles model the depolarization and repolarization as if they were permanent.

The results of the simulations that were performed using the Fig. 3 model are illustrated in Fig. 4, which is a schematic diagram illustrating an example of a) the simulated magnetic flux density and b) corresponding streamlines, around a nerve fiber, in accordance with some aspects of the present disclosure. Fig. 4 represents a snapshot at a specific time at a fixed position next to the nerve. The simulated magnetic flux density illustrated in Fig. 4 has an axisymmetric magnetic field around the nerve fiber.

In practice, however, both the electrical impulse generated by a nerve's activation, and the magnetic field, propagate along the nerve fiber. Moreover, the magnetic field illustrated in Fig. 4 only represents the magnetic field generated by a single nerve fiber. These shortcomings of the model were captured in a more-detailed model.

In the more-detailed model, the propagation of the magnetic field was modelled by assigning to the pair of opposing dipoles a constant propagation velocity v. An electromotive force was also prescribed inside the dipole in order to generate a local current ranging between 50 and 100 nA. Moreover, in practice, a bundle of nerves includes thousands of fibers. Although the activation of the nerves within the bundle is synchronized, differences between the nerve fibers within the bundle results in a dynamic magnetic field signal due to the propagation of the action potential and due to the different arrival time respective to each fiber. In the more-detailed model, the effect of a bundle of nerve fibers was modelled using a Gaussian distribution for the arrival times of the magnetic field signals for the fibers in the bundle.

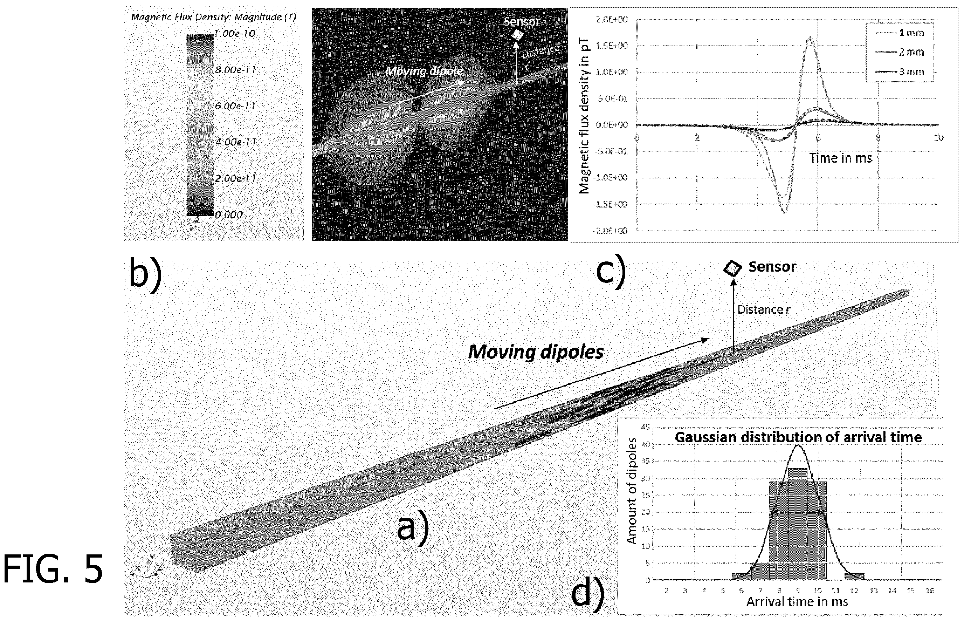

The results of simulations that were performed with this more-detailed model are illustrated in Fig. 5, which is a diagram illustrating an example of a) a model of a dipole current in a nerve fiber with a sensor located at radial distance $r$, b) a predicted magnetic flux density arising from the modelled dipole current, c) the predicted magnetic flux density at various radial distances, $r$, and d) the distribution of the arrival times of the magnetic flux density used to generate the predicted magnetic flux density in c), in accordance with some aspects of the present disclosure. The magnetic flux density signal illustrated in Fig. 5c) has an amplitude of up to approximately 1.5pT, depending on the radial distance between the sensor and the nerve bundle. This is consistent with the typical magnetic field radial decrease around dipoles reported in literature. Moreover, it follows a $1/r^n$ power law, with $n$ in the range from approximately 1 to 3, depending on the distance, the type of nerve and the number of fibers. The simulations illustrate that a measurement of the magnetic field generated by a renal nerve may be performed using a magnetic sensor having a sensitivity of a few picoTesla if the sensor is located within a few millimeters of the nerve bundle. The results of this more-detailed model were then used to design a magnetic sensor for detecting nerve activity.

Various examples of an interventional device that includes a magnetic sensor for detecting nerve activity are described below. The magnetic sensor may be used to measure the magnetic fields that are produced by nerve activity, such as the activity of a renal nerve, as described above with reference to Fig. 1. In general, the magnetic sensor that is disposed on the interventional device may be used to measure the magnetic fields that are generated in response to an artificial activation of a nerve, or it may be used to measure the magnetic fields that are generated by a natural, i.e. a spontaneous, activation of a nerve.

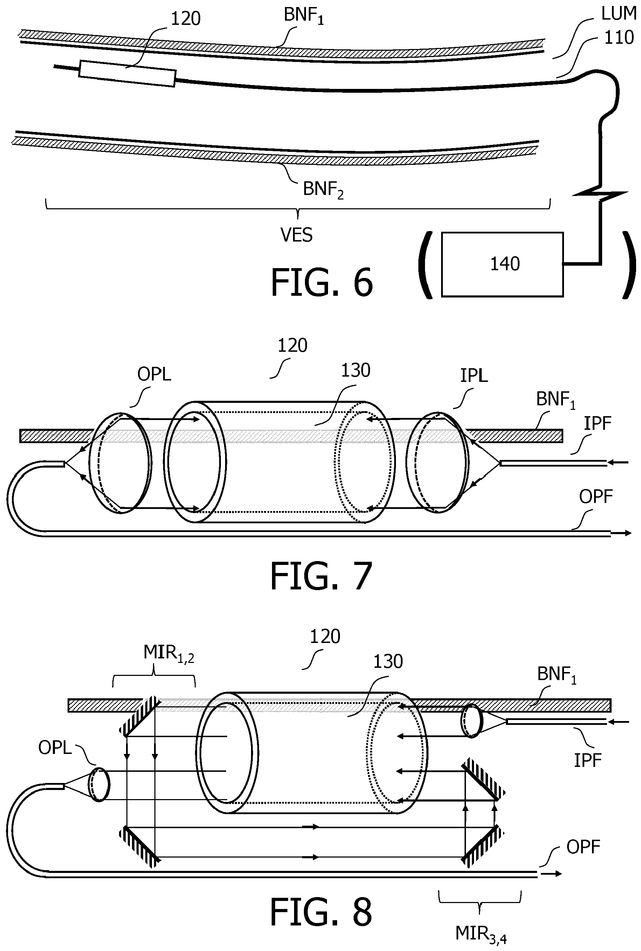

Fig. 6 is a schematic diagram illustrating an example of an interventional device 110 for detecting nerve activity, in accordance with some aspects of the present disclosure. In the illustrated example, the interventional device includes a magnetic sensor 120. The magnetic sensor 120 is coupled to an insertable portion of the interventional device, i.e. insertable into a body lumen such as for example a renal artery. The magnetic sensor 120 is configured to generate signals in response to magnetic fields produced by nerve activity.

With reference to Fig. 6, in this example, the interventional device 110 is a catheter. The catheter includes an insertable portion that is inserted into a lumen LUM of the blood vessel VES illustrated in Fig. 6. Thus, in this example, the insertable portion of the interventional device is sized for insertion into a blood vessel. The magnetic sensor 120 may in general be coupled to any location on the insertable portion of the interventional device. For example, the magnetic sensor 120 may be coupled to the distal end of the insertable portion, or to a location that is proximal with respect to the distal end of the insertable portion. In the illustrated example, the magnetic sensor 120 is coupled to the interventional device at a location that is proximal with respect to a distal end of the insertable portion of the catheter. In general, the magnetic sensor 120 may be coupled to any location on the insertable portion of an interventional device, and the interventional device may be insertable into any region of the anatomy.

With continued reference to Fig. 6, the magnetic sensor 120 generates signals in response to magnetic fields produced by nerve activity. The signals may be transmitted to a controller, such as the controller 140 illustrated in Fig. 6. In general, the generated signals may be optical signals or electrical signals. For instance, in implementations described below in which the magnetic sensor is provided by an optically pumped magnetometer "OPM", the generated signals are optical signals, and these are transmitted optically to the controller 140 via the optical fiber(s) that deliver optical irradiation to an optical cell of the OPM. Transmitting the signals optically avoids the risk of generating magnetic fields that may interfere with the operation of the magnetic sensor 120. The signals may alternatively be transmitted wirelessly. The controller 140 may process the signals in order to output a detection result indicative of nerve activity, as described in more detail below.

The magnetic fields that are sensed by the magnetic sensor 120 may be generated by nerves such as the bundle of nerve fibers BNFi illustrated in Fig. 6. In general, the nerve activity may be triggered artificially, or it may be triggered naturally, i.e. spontaneously. In the former case, the nerve activity may be triggered by applying a stimulus within the vessel VES, i.e. from an intravascular position, or more generally from an intra-corporeal position, or alternatively it may be triggered by applying a stimulus from a position outside the body, i.e. from an extra-corporeal position. The nerve activity may be triggered using various techniques, including by the delivery of electrical energy, RF energy, thermal energy, and tactile stimulation. In one example, the interventional device is an intravascular RF ablation device, and the nerve activity is stimulated by means of RF energy to the nerve from the intravascular RF ablation device.

With continued reference to Fig. 6, the magnetic sensor may be disposed within the vessel VES such that the magnetic sensor is adjacent to, or even touches, the wall of the vessel VES in order to detect the magnetic field generated by the bundle of nerve fibers BNFi. This magnetic field decays rapidly with separation between the nerve fibers and the magnetic sensor, and so with the magnetic sensor 120 is in the illustrated position, it may detect a much weaker contribution from the bundle of nerve fibers $BNF_2$ than from the bundle of nerve fibers BNFi.

The magnetic sensor 120 illustrated in Fig. 6 may be provided by various types of magnetic sensor. For example, the magnetic sensor 120 may be provided by an optically pumped magnetometer "OPM", as mentioned above. Fig. 7 is a schematic diagram illustrating a first example of a magnetic sensor 120 that includes an optically pumped magnetometer, in accordance with some aspects of the present disclosure. With reference to Fig. 7, the OPM comprises an optical cell 130 containing an alkali metal in a liquid and/or a gaseous phase. The alkali metal may be Rubidium, Caesium, or Potassium, for example. The OPM is configured to generate the signals in response to magnetic fields produced by nerve activity by measuring a magnetic-field-dependent optical property of the alkali metal. The magnetic-field-dependent optical property may be a direction of atomic spin of the alkali metal, for example.

With reference to Fig. 7, the example optical cell 130 includes a tubular member that is bounded by optical windows at its axial ends, and which in combination with the tubular member serve to contain the alkali metal in its liquid and/or gaseous phase. By way of an example, the optical cell 130 illustrated in Fig. 7 may have a diameter of approximately 1 millimeter, or less, in order that the optical cell 130 may be coupled to an interventional device and inserted into a subject. In some examples, the optical cell 130 has a diameter of approximately 0.5 millimeter, or less, a length of approximately 3 millimeters, and a resulting volume of 1 cubic millimeter, or less. A bundle of nerve fibers BNFi is also illustrated in Fig. 7. As described above with reference to Fig. 4, nerve activity in the bundle of nerve fibers BNFi generates magnetic fields. In order to detect the magnetic fields that are generated by the bundle of nerve fibers BNFi, optical irradiation is inputted to the optical cell 130 via an input optical fiber IPF and an input lens IPL. The optical irradiation irradiates the alkali metal in its liquid and/or gaseous phase in the optical cell 130. The optical irradiation may be provided by a laser (not illustrated in Fig. 7) that has a wavelength that corresponds to a resonance frequency of the atoms of the alkali metal. The laser may be controlled by the controller 140 illustrated in Fig. 6. The laser provides a polarised beam that passes through the optical cell 130. In some examples, the beam may have a circular polarisation. At resonance, the atomic spin of the alkali metal atoms in the optical cell 130 is aligned with the direction of propagation of the laser radiation. Changes in the magnetic field in the vicinity of the optical cell deflect the atomic spin direction, which in turn cause changes in the amount of radiation that is transmitted through the optical cell 130. In other words, a direction of the spin of the atoms of the alkali metal is susceptible to magnetic fields. In the example illustrated in Fig. 7, the radiation that is transmitted through the optical cell is collected by an output lens OPL and coupled to an optical detector (not illustrated in Fig. 7) via an output optical fiber OPF. A polarizer may be included within the optical path in order to measure the transmission for a particular polarization. In one example, the optical detector may be provided by a polarimeter. The optical detector measures the amount of radiation that is transmitted through the optical cell 130 with a particular polarization, and thereby determines the strength of the magnetic field in the vicinity of the optical cell in a specific plane.

Variations of the above-described implementation of an OPM, are also contemplated.

In one example, the optical cell 130 of the OPM further contains a background gas. In this example, the OPM is further configured to generate the signals in response to magnetic fields produced by nerve activity by measuring a magnetic-field-dependent optical property of the background gas. The background gas may include Helium, for example. In this example, the direction of the spin of atoms of the background gas, e.g. Helium, is also susceptible to changes in magnetic field. Magnetic fields that are produced by nerve activity may be detected via changes in the spin of Helium atoms in the optical cell 130 in a similar manner to that described above for alkali metal atoms. In other words, by irradiating an optical cell 130 that includes Helium gas as well as an alkali metal in the liquid and/or gaseous phase, changes in the transmission of the optical cell that arise from a deflection of the atomic spin direction of both the Helium gas as well as the alkali metal may be measured in order to detect magnetic fields. A different wavelength of laser radiation may be used to induce resonance in the Helium atoms than that of the alkali metal atoms. This permits the transmission measurements for the Helium and the alkali metal to be performed separately.

By using the models described above with reference to Fig. 2 - Fig. 5, the inventors have determined that the detection of magnetic fields that are generated by a renal nerve from a position within the renal artery can be achieved by positioning a magnetic sensor with sub-picoTesla sensitivity a few millimeters from a bundle of nerve fibers. The inventors have also determined that the magnetic field sensitivity of an OPM cell is determined by the density of atoms per unit volume in the gaseous phase in the cell. The magnetic field sensitivity of an OPM cell may be described by the Equation:

$$Resolution\ (picoTesla) \propto \frac{1}{\sqrt{n \cdot V}}$$

wherein n represents the density of atoms per unit volume in the gaseous phase in the cell, and V represents the volume of the cell. In this equation, a lower value for the Resolution corresponds to a more-sensitive OPM cell. Moreover, to allow a free spinning in a proper quantum state, the atoms must be in gaseous phase. For alkali metals such as Rubidium, the boiling point is above room temperature. Thus, the optical cell must be heated to reach the gaseous phase. Above the boiling temperature, the density of atoms per unit volume in the gaseous phase in the cell, $n$, is significantly dependent on temperature. A further increase in temperature of the cell results in a larger density $n$, which increases the sensitivity of the OPM . By contrast, Helium has a boiling point that is well below room, or body temperature, and as a consequence, substantially all of the Helium atoms are in a gaseous phase at these temperatures. As a consequence, magnetic fields may be detected via changes in the spin of Helium atoms at room, or body temperature. Heating has little effect on the sensitivity of an optical cell that includes Helium. The pressure can be increased in an optical cell that contains Helium to increase the density of atoms per unit volume in the gaseous phase, $n$, assuming that the Helium remains in the gaseous phase. However, $n$ remains lower than for heated alkali metals such as Rubidium, Caesium or Potassium, and consequently the expected sensitivity of the OPM with Helium will be lower than that of an OPM that contains alkali metals.

In general, a higher atom density, $n$, increases the relaxation time of the atoms transition between quantum states. Thus, the bandwidth of the OPM is inversely proportional to the atom density, $n$. For a sufficient bandwidth of the device, there is consequently an upper limit in the atoms density in the optical cell.

By providing an OPM with an optical cell that includes both a background gas such as Helium, as well as an alkali metal in a liquid and/or a gaseous phase, an OPM may be provided that can operate in a relatively lower sensitivity mode in which the optical cell is not heated, and in which the magnetic fields produced by nerve activity are detected via changes in a spin of atoms of the background gas, e.g. Helium, and also in a relatively higher sensitivity mode in which the optical cell is heated, and the magnetic fields produced by nerve activity are detected via changes in a spin of atoms of the alkali metal.

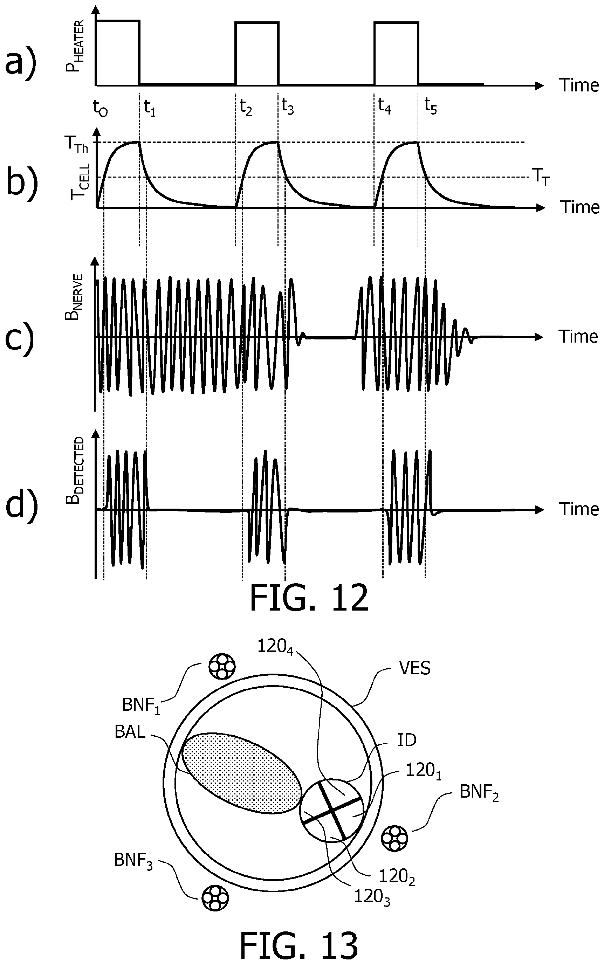

Thus, in some examples, the OPM described with reference to Fig. 7 also includes a heater, and the heater is configured to supply heat to the optical cell. In this respect, the use of various types of heater is contemplated. The heater may for example include a resistive element that is thermally coupled to a housing of the optical cell 130. In this example, electrical energy may be delivered to the resistive element via electrical wires that extend along a length of the interventional device to the controller 140. In order to avoid the risk of interference between magnetic fields generated by currents in a resistive heater, and the magnetic sensor 120, the heater may be operated in a pulsed mode, described later with reference to Fig. 12, and wherein magnetic fields are sensed by the magnetic sensor 120 during a time period after a supply of electrical energy to the heater has been switched off. Alternatively, the heater may be provided by an optical absorber that is thermally coupled to a housing of the optical cell. In this example, an optical source, which may for example be the same laser that is used to irradiate the optical cell, or a second laser emitting a different optical wavelength to a wavelength of the laser that irradiates the optical cell, is coupled to the optical absorber. The optical absorber is configured to absorb an optical wavelength that is emitted by the optical source. If the same laser is used to irradiate the optical cell as well as to heat the optical cell, the input optical fiber IPF may deliver optical irradiation from the laser to a beamsplitter, which directs a first portion of the irradiation from the laser to the optical absorber and a second portion of the irradiation from the laser to the optical cell. If a second laser is used to heat the optical cell, a second optical fiber may be used to couple the irradiation from the second laser to the optical absorber. The use of a second laser isolates the operation of heating of the optical cell from the operation of detecting the magnetic field via changes in the spin direction of the atoms in the optical cell. The use of an optical absorber in combination with such optical sources may provide a heater with a smaller geometry than a resistive heater, and also reduces the risk of generating magnetic fields that may interfere with the magnetic sensor 120.

The inventors have also determined that whilst significant improvements in the sensitivity of the OPM described above may be achieved by heating the optical cell, as described above, these improvements are at the expense of the need to thermally insulate the OPM, or to remove heat dissipated by the OPM in order to prevent damage to tissue. For instance, in one design, at a temperature of 50 degrees centigrade, an OPM cell may achieve a sensitivity of approximately 150 femtoTesla, whereas by heating the OPM cell to a temperature of approximately 100 degrees centigrade, an OPM cell may achieve a five-fold improvement in sensitivity to approximately 30 femtoTesla. In order to achieve such optical cell temperatures in an interventional device without damaging tissue, in one approach, thermal insulation may be provided in order to limit the rate of thermal energy escaping the OPM, and thereby limit the temperature increase of an outer surface of the OPM. In another approach, which may be combined with the former approach, heat that is dissipated by the OPM may be removed from the OPM using a cooling arrangement. In this regard, the use of various cooling arrangements is contemplated.. In some of these cooling arrangements, the medium in which the interventional device is inserted is used to remove the heat that is dissipated. In one example, the OPM comprises a housing configured to provide thermal contact between the OPM and a medium in which the interventional device is inserted. In this example, heat is removed from the OPM by the medium. If the medium is blood, for example, the flowing blood may be used to remove a significant amount of heat. The thermal contact may be provided by forming the housing of the OPM from various thermally conductive materials, such as metals, and which may form a thermal path between the OPM housing and the medium. In a related example, which may be used in combination with the former example, the OPM is in thermal contact with an outer surface of the interventional device 110 for cooling the OPM via the outer surface of the interventional device. By providing such thermal contact with the interventional device, heat is dissipated via the interventional device, which limits the temperature of the OPM, thereby reducing the risk of damage to tissue that is in thermal contact with the OPM. In another example, which may also be used in combination with either of the former examples, the interventional device includes a channel that is configured to supply a cooling fluid to the OPM. The channel may extend along a length of the interventional device such that the cooling fluid may be supplied to the OPM from a position that is external to the subject. The channel may be coupled to a source of cooling fluid, such as an open-circuit source of cooling fluid, or a closed-circuit heat exchanger. The cooling fluid may include water, or saline, for example.

In these examples that include a heater, the interventional device may also include a temperature sensor that is in thermal contact with i) an outer surface of the interventional device, or ii) the optical cell 130. The temperature sensor may be used to monitor, and as described later, to control, a temperature of the outer surface of the interventional device, or the optical cell 130, respectively.

The sensitivity of the OPM described with reference to Fig. 7 may also be improved by measuring the transmission of an optical beam that has passed through the optical cell 130 multiple times. In this case, each pass of the optical beam has an incremental effect on the measured transmission. Thus, in one example, the OPM is configured to measure a transmission of an optical beam passing through the optical cell 130 in order to generate the signals in response to magnetic fields produced by nerve activity; and the OPM comprises a plurality of optical elements configured to provide multiple passes of the optical beam through the optical cell 130.

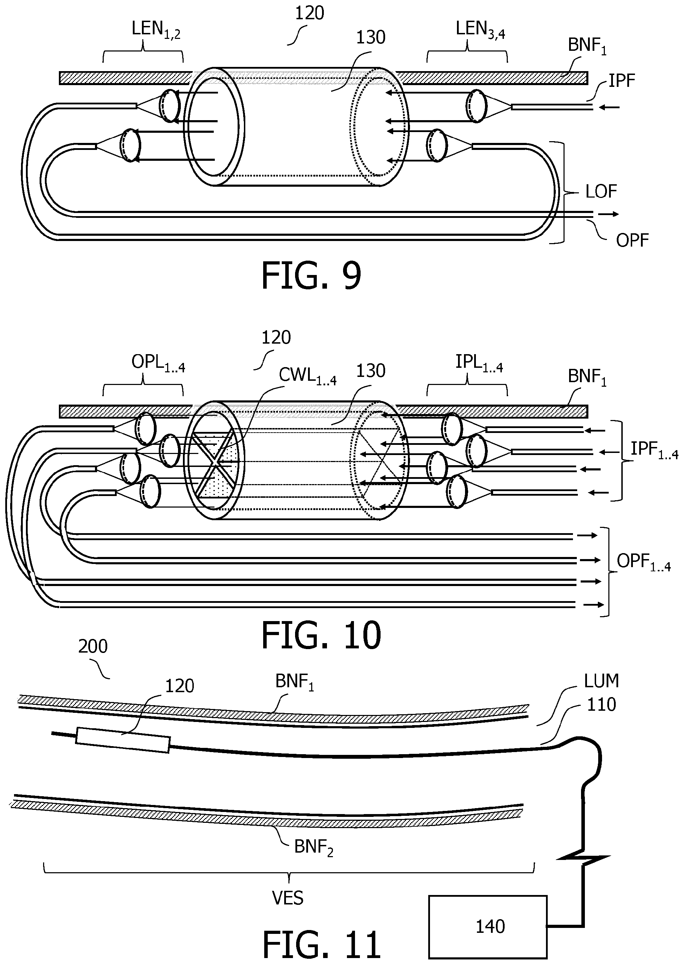

In this example, the optical elements may be provided by reflective elements and/or lenses in combination with optical fibers. Two implementations of this example are described with reference to Fig. 8 and Fig. 9. Fig. 8 is a schematic diagram illustrating a second example of a magnetic sensor 120 that includes an optically pumped magnetometer, in accordance with some aspects of the present disclosure. The example illustrated in Fig. 8 includes elements that are also represented in Fig. 7. Elements in Fig. 8 that have the same labels as Fig. 7 provide the same function as described with reference to Fig. 7. In comparison to Fig. 7, the Fig. 8 example additionally includes a plurality of reflective elements, $MIR_{1..4}$, and which are arranged to provide multiple passes of the optical beam that irradiates the optical cell, through the optical cell 130. Fig. 9 is a schematic diagram illustrating a third example of a magnetic sensor 120 that includes an optically pumped magnetometer, in accordance with some aspects of the present disclosure. The example illustrated in Fig. 9 includes elements that are also represented in Fig. 7. Elements in Fig. 9 that have the same labels as Fig. 7 provide the same function as described with reference to Fig. 7. In comparison to Fig. 7, the Fig. 9 example additionally includes a plurality of lenses $LEN_{1..4}$, and an optical fiber LOF, and which are arranged to provide multiple passes of the optical beam that irradiates the optical cell, through the optical cell 130.

The inventors have also observed that a drawback of some existing magnetic sensors is their inability to provide information on the location of nerves contributing most to the detected magnetic field level. As may be appreciated from Fig. 1, a magnetic sensor that is disposed on an interventional device ID in the illustrated position may detect the magnetic fields that are generated by the bundle of nerve fibers BNFi. However, the signals generated by such a sensor provide little information on the direction in which the bundle of nerve fibers BNFi is located. A physician only knows the direction in which a bundle of nerve fibers BNFi is located by moving the magnetic sensor towards the bundle of nerve fibers and observing an associated increase in detected magnetic field. If the direction of the bundle of nerve fibers BNFi were known before moving the magnetic sensor it would help the physician with positioning the magnetic sensor closer to the bundle of nerve fibers.

In one example, the magnetic sensor 120 includes a plurality of magnetic sensor elements, and each magnetic sensor element is configured to detect magnetic fields intercepting a different volume of the interventional device.

This example may be used to improve the positioning of a sensor with respect to a bundle of nerve fibers. This example is described with reference to Fig. 10, which is a schematic diagram illustrating a fourth example of a magnetic sensor 120 that includes an optically pumped magnetometer, in accordance with some aspects of the present disclosure. The example illustrated in Fig. 9 includes elements that are also represented in Fig. 7. Elements in Fig. 10 that have the same labels as Fig. 7 provide the same function as described with reference to Fig. 7. In Fig. 10, there are four magnetic sensor elements. Each magnetic sensor element includes an input lens $IPL_{1..4}$ and an input optical fiber $IPF_{1..4}$ that are configured to couple optical irradiation to a portion of the optical cell 130, and a corresponding output lens

$OPL_{1..4}$ and an output optical fiber that are configured to collect optical irradiation from the portion of the optical cell and to couple the collected optical irradiation to an optical detector. Each portion of the optical cell represents a sensing volume within which magnetic fields are sensed by a magnetic sensor element. The portions of the optical cell are defined by cell walls, $CWL_{1..4}$. The cell walls confine the gas in the optical cell to discrete sensing volumes. The strength of the magnetic field intercepting each sensing volume is determined by measuring the transmission of the optical irradiation through the respective portion of the optical cell in the manner that was described in relation to Fig. 7. The OPM 120 illustrated in Fig. 10 is also coupled to an interventional device as described above. In so doing, each of the magnetic sensor elements may be used to detect magnetic fields intercepting a different volume of the interventional device.

In the above example, the measurement of the magnetic field strength in each of the different volumes of the interventional device provides information on a user on how to reposition the interventional device so as to move it towards a nerve. For instance, in the example illustrated in Fig. 10, since the uppermost optical path is closest to the bundle of nerve fibers BNFi, the magnetic field that is measured in this optical path as a result of nerve activity in the bundle of nerve fibers BNFi, will be higher than the magnetic field that is measured in the lower optical path in Fig. 10. Thus, a direction in which a nerve is located may be determined based on a comparison of the values of the magnetic field strengths that are measured in the different volumes of the interventional device.

In another example, the interventional device described with reference to Fig. 6 also includes an expandable balloon or basket. The balloon or basket is coupled to the insertable portion of the interventional device for immobilizing the inserted portion of the interventional device in the lumen when the expandable balloon or basket is in an expanded state. This example may be used to provide magnetic field measurements at a fixed position in the lumen, without e.g. the position of the interventional being affected by subject motion, cardiac motion, blood flow, and so forth.

The examples interventional devices described above may also form part of a system 200 that includes a controller 140. The controller 140 receives signals generated by the magnetic sensor, and outputs a detection result indicative of nerve activity in response to the received signals.

Fig. 11 is a schematic diagram illustrating an example of a system 200 that includes an interventional device 110 for detecting nerve activity, and a controller 140, in accordance with some aspects of the present disclosure. The example illustrated in Fig. 11 includes elements that are also represented in Fig. 6. In Fig. 11, the system 200 includes the interventional device 110 that was described in relation to Fig. 6, and also a controller 140. Elements in Fig. 11 that have the same labels as Fig. 6, such as the interventional device 220, provide the same function as was described above with reference to Fig. 6. The controller 140 illustrated in Fig. 11 may include one or more processors and/or electronic circuitry. The one or more processors and/or electronic circuitry are configured to perform the various operations that are described in relation to the controller. For instance the controller may include an optical detector and electronic circuitry that respectively detect, and amplify the received signals. The controller may also include an analogue-to-digital converter "ADC" that digitises the signals, and a processor that converts the digitised signals and/or performs further operations on the digitised signals, such that they may be displayed as a detection result on a display device such as a monitor.

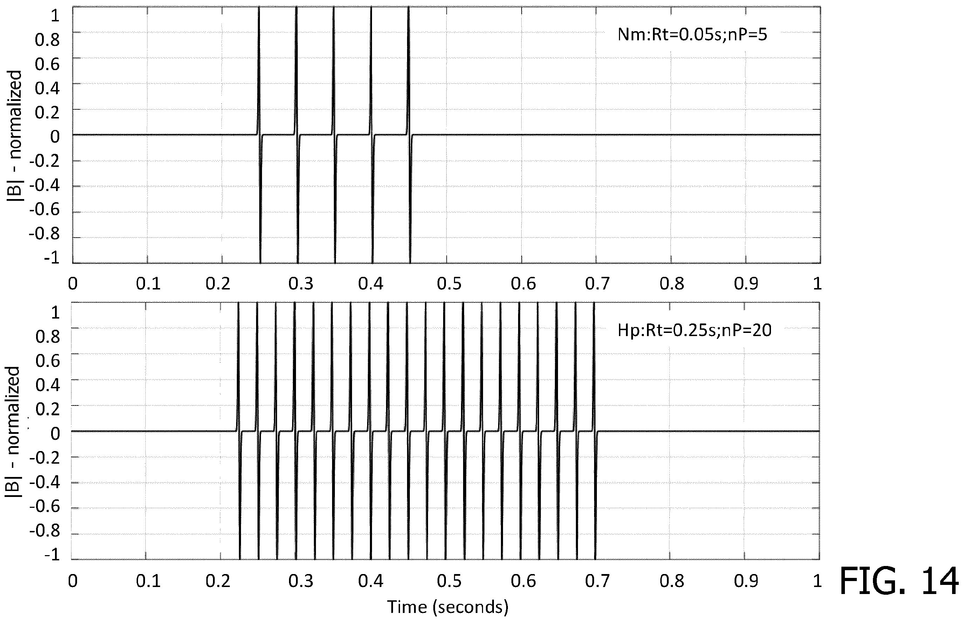

Various detection results may be outputted by the controller 140. In one example, the detection result includes a time-dependent signal that represents the signals generated by the magnetic sensor 120, or in other words, the time-dependent magnetic fields that are produced by the nerve activity. In another example, the detection result includes a signal representing a magnitude of the signals generated by the magnetic sensor 120, or in other words, the magnitude of the magnetic fields that are produced by the nerve activity. In another example, the detection result includes an estimate of the separation between the magnetic sensor 120 and a nerve that produced the nerve activity. An estimate of the separation between the magnetic sensor 120 and a nerve may be determined based on the magnitude of the signals that are generated by the magnetic sensor. The model described above with reference to Fig. 2 Fig. 5 may be used to provide a lookup table that associates the magnitude of the signals to the separation, r, in Fig. 5. In another example, the detection result includes an indication of a status of a nerve (e.g. healthy, unhealthy) that produced the nerve activity. In another example, the detection result includes a measure of sympathetic overdrive in a nerve that produced the nerve activity. In another example, the detection result includes a suitability for the nerve that produced the nerve activity to be treated by renal denervation therapy. The suitability may be assessed by determining, based on the detected magnetic field strength, a distance between the magnetic sensor 120 and the nerve caused the detected nerve activity. If the distance is too large, the cell wall might be damaged by activating an ablation device and/or the nerve may not be ablated sufficiently. Alternatively or additionally, the suitability may be assessed based on the signature of the detected signals, as described later with reference to Fig. 14. For instance, the magnitude of sympathetic overdrive may be determined from the signature, this magnitude used to determine the suitability of the nerve for treatment by renal denervation therapy. In another example, the detection result includes a measure of the effectiveness of a renal denervation treatment procedure that has been performed on the nerve that produced the nerve activity. The measure of the effectiveness of a renal denervation treatment procedure may be determined based on a comparison of the signals generated by the magnetic sensor prior to, and after the renal denervation treatment procedure. As the treatment progresses, the amplitude of the detected magnetic signals decreases until the treated nerve is destroyed. Moreover, as described later with reference to Fig. 14, the signature of the pulses of magnetic fields generated by a nerve, i.e. the frequency of pulses in a pulse train, and the number of pulses in a pulse train, may also change during treatment. A measure of the effectiveness of the treatment may therefore be determined by measuring the amplitude of the pulses and/ or the signature. A user of the system may also be informed that treatment may be terminated when a desired level of treatment effectiveness has been reached, i.e. that the amplitude of the pulses and/ or the signature has reached a desired state. Other detection results may also be outputted by the controller 140. The detection result(s) may be outputted in various ways, such as to a computer readable storage medium, to a printer, or to a display device such as a monitor, and so forth.

In one example of the system 200 described with reference to Fig. 11, the interventional device may be translated along an axis of a lumen whilst generating the detection result(s) in order to provide a 2D or 3D map of the detection result(s). The interventional device may be translated using various manipulators. The changes of the magnetic field strength and direction along a scan path from nerves are expected to be reproducible. Consequently, by translating the interventional device along the axis of the lumen multiple times, and generating corresponding maps, detected features that do not appear in multiple maps may be identified as noise, or interference, and discarded. A combined map may then be generated from the individual maps by selecting, and e.g. averaging, the maps that do not contain the identified noise or interference, or by combining the maps from which the interference has been removed. The 2D or 3D map of the detection result(s) may be outputted by the controller 140.

As described above in relation to the interventional device 110, in some examples the magnetic sensor 120 comprises an OPM, and the OPM further comprises a heater that is configured to supply heat to the optical cell 130. In one example of the system 200 described with reference to Fig. 11, the controller 140 may be configured to selectively operate the OPM in a relatively lower sensitivity mode and in which a relatively lower power level is supplied by the heater to the optical cell 130, and also in a relatively higher sensitivity mode and in which a relatively higher power level is supplied by the heater to the optical cell 130, in order to generate the signals in response to magnetic fields produced by nerve activity.

In this example, the optical cell 130 may contain one type of alkali metal in a liquid and/or a gaseous phase. The alkali metal may be Rubidium, for example.

The two levels of sensitivity that are provided in this manner may be used to e.g. firstly identify the location of a bundle of nerve fibers in the relatively lower sensitivity mode, and after having identified the bundle of nerve fibers, to switch the OPM into operation in the relatively higher sensitivity mode in order to perform a quantified analysis of the identified bundle of nerve fibers. In this example, the relatively lower power level may be negligible, or zero power, for example. In this example, changes in the magnetic field in the vicinity of the optical cell are detected via changes in the magnetic-field-dependent optical property, i.e. the atomic spin direction, of the atoms of the same alkali metal in both the relatively lower sensitivity mode, and also in the relatively higher sensitivity mode. By operating the OPM in this manner, a temperature, or a power consumption of the OPM may be reduced because the OPM consumes lower power when a relatively lower power is applied to the heater. Limiting the temperature or power consumption in this manner also limits the risk of the OPM damaging tissue. In this example, the controller may automatically switch between the relatively lower sensitivity mode and the relatively higher sensitivity mode in response to the detection of magnetic fields in the relatively lower sensitivity mode above a predetermined threshold.

Alternatively, in this example, the optical cell 130 may instead contain two different types of alkali metals in a liquid and/or a gaseous phase, e.g. Rubidium and Caesium, or the optical cell 130 may contain one type of alkali metal in a liquid and/or a gaseous phase and also a background gas, such as Helium. The two types of alkali metals, or the one type of alkali metal and the background gas, may then be used in a similar manner to provide the relatively lower sensitivity mode, and the relatively higher sensitivity mode. In this example, changes in the magnetic field in the vicinity of the optical cell are detected via changes in the magnetic-field-dependent optical property, i.e. the atomic spin direction, of the atoms of different materials, i.e. different alkali metals, or the background gas and an alkali metal, in each of the relatively lower sensitivity mode and the relatively higher sensitivity modes.

In these examples, the optical cell 130 contains an alkali metal in a liquid and/or a gaseous phase, and a background gas; and the OPM is configured to generate the signals in response to magnetic fields produced by nerve activity by measuring a magnetic-field-dependent optical property of the alkali metal and the background gas, and in the relatively lower sensitivity mode an optical property of the background gas is sensed, and in the relatively higher sensitivity mode an optical property of the alkali metal is sensed; or alternatively: the optical cell 130 contains a first alkali metal in a liquid and/or a gaseous phase, and a second alkali metal in a liquid and/or a gaseous phase; and the OPM is configured to generate the signals in response to magnetic fields produced by nerve activity by measuring a magnetic-field-dependent optical property of the first alkali metal and the second alkali metal, and in the relatively lower sensitivity mode an optical property of the first alkali metal is sensed, and in the relatively higher sensitivity mode an optical property of the second alkali metal is sensed.

As mentioned above, in some examples, the OPM includes a heater that is configured to supply heat to the optical cell 130. In these examples, the interventional device may also include a temperature sensor. The temperature sensor may be in thermal contact with i) an outer surface of the interventional device, or ii) the optical cell 130. In one example of the system 200 described with reference to Fig. 11, the controller 140 is configured to operate the heater in response to a temperature measured by the temperature sensor. For instance, the heater may be switched on until the temperature sensed by the sensor reaches a predetermined value. At this time, the heater may be switched off. The temperature of the heater then falls as a consequence of thermal diffusion, and/or active cooling, following which the heating cycle is repeated. The controller 140 may alternatively or additionally be configured to control the cooling arrangement described above in response to the temperature measured by the temperature sensor. For instance, the cooling arrangement may be activated if a temperature measured by the temperature sensor exceeds a predetermined threshold.

By operating the heater in response to a temperature measured by the temperature sensor, the risk of damage to tissue may be reduced. The controller may operate the heater in a continuous mode, or in a pulsed mode. When operated in a continuous mode, the controller may operate the heater in a digital manner such that if the temperature measured by the temperature sensor is below a desired temperature threshold, the heater switched on, and such that if the temperature measured by the temperature sensor reaches the desired temperature threshold, the heater is switched off. The controller may alternatively operate the heater in a continuous mode, and in an analogue manner by using a proportional-integral-derivative controller "PID" feedback loop.

The controller may alternatively operate the heater in a pulsed mode, and use the temperature that is measured by the temperature sensor to determine when to switch off the heater. An example of the operation of the heater in a pulsed mode is described with reference to Fig. 12, which is a schematic diagram illustrating examples of a) a magnitude of a power $P_{HEATER}$ applied to a heater of an optical cell of an OPM, b) a resulting temperature $T_{CELL}$ of the optical cell, c) a magnetic field $B_{NERVE}$ generated by an activated nerve, and d) a signal $B_{DETECTED}$ generated by the OPM in response to a detection of the magnetic field $B_{NERVE}$, in accordance with some aspects of the present disclosure.

Fig. 12 illustrates in a) a magnitude of a power $P_{HEATER}$ applied to a heater of an optical cell of an OPM. The power $P_{HEATER}$ is applied in a pulsed mode in order to limit the amount of power dissipated into a medium in which the OPM is inserted, and also to limit the power consumption of the OPM. A constant level of power is applied to the heater in the intervals to - $t_1$, $t_2$ - $t_3$, and $t_4$ - $t_5$. The resulting temperature $T_{CELL}$ of the optical cell is illustrated in Fig. 12b. This temperature may be measured by a temperature sensor that is in thermal contact with the optical cell 130. Alternatively, this temperature may be measured by a temperature sensor that is in thermal contact an outer surface of the interventional device. In the illustrated example, the heater applies heat to the optical cell periodically. The heater power is switched on at the times $t_0$, $t_2$, and $t_4$. During the intervals when power is applied to the heater, i.e. to - $t_1$, $t_2$ - $t_3$, and $t_4$ - $t_5$, the temperature of the cell increases until a threshold temperature $T_{Th}$ is reached. When the temperature of the cell $T_{CELL}$ reaches the threshold temperature $T_{Th}$, the power $P_{HEATER}$ that is applied to the heater is switched off. In the intervals when power is not applied to the heater, i.e. $t_1$ - $t_2$, and $t_3$ - $t_4$, the temperature of the cell $T_{CELL}$ decreases due to heat loss into the medium in which the OPM is inserted, or due to heat loss into a cooling fluid, as described above. Fig. 12c illustrates a magnetic field $B_{NERVE}$ generated by an activated nerve. In this example, the nerve is activates spontaneously, and includes a first set of pulses that overlap the interval between to and $t_3$, and the interval between $t_4$ and $t_5$. As seen in Fig. 12d, the magnitude of the signal $B_{DETECTED}$ generated by the OPM in response to a detection of the magnetic field $B_{NERVE}$, in Fig. 12c, is higher in time periods that correspond to the intervals to - $t_1$, $t_2$ -$t_3$, and $t_4$ - $t_5$ than outside these intervals. This is due to the heating of the optical cell of the OPM, and the consequent increase in sensitivity of the OPM in these intervals. In practice, and as illustrated in Fig. 12d, the signal $B_{DETECTED}$ generated by the OPM is delayed as compared to the intervals to $t_1$, $t_2$ - $t_3$, and $t_4$ - $t_5$ when power is applied to the heater. This delay is caused by the time lag between the time at which heat is applied to the heater, and the resulting cell temperature $T_{CELL}$. As illustrated in Fig. 12d, in some cases the increase in sensitivity may only occur when the cell temperature $T_{CELL}$ exceeds a particular temperature $T_T$. Due to the time lag in the cell temperature behind the application of power to the heater, the signal $B_{DETECTED}$ generated by the OPM may therefore lag behind the signal $P_{HEATER}$, as illustrated in Fig. 12. Thus, in the illustrated example, at least some of the spontaneous activation of the nerve in c), is detected. As may be appreciated, by adjusting the frequency at which the power $P_{HEATER}$ is applied to the heater in Fig. 12a, and the threshold temperature $T_{Th}$, a desired power consumption, cell temperature, and duration of the sensing period, may be achieved. Instead of operating in the pulsed mode illustrated in Fig. 12, the OPM may alternatively be operated in a continuous mode as described above, and wherein the applied power $P_{HEATER}$ is adjusted continuously over all periods in response to the measured temperature of the cell $T_{CELL}$.

As described above in relation to the interventional device 110, in some examples the magnetic sensor 120 comprises an optically pumped magnetometer, OPM, and the OPM comprises an optical cell 130 containing an alkali metal in a liquid and/or a gaseous phase, and the OPM is configured to generate the signals in response to magnetic fields produced by nerve activity by measuring a magnetic-field-dependent optical property of the alkali metal. In one example of the system 200 described with reference to Fig. 11, the OPM includes an optical source configured to provide an optical pump beam, and the OPM is configured to measure the magnetic field-dependent optical property of the alkali metal in response to an excitation of the alkali metal within the optical cell 130 by the optical pump beam, and the controller 140 is further configured to:

- modulate an intensity of the optical pump beam between a relatively lower intensity and a relatively higher intensity; and
- the OPM is configured to measure the magnetic field-dependent optical property of the alkali metal by determining a transmission of the optical pump beam through the optical cell 130 whilst the optical pump beam excites the alkali metal at the relative higher intensity.

In this example, rather than operating the optical source of the OPM in a continuous mode, the optical source is operated in a pulsed mode. This has the effect of allowing the system 200 to be operated at a reduced level of power. The relatively lower intensity may be negligible, or zero intensity. This example, may also be performed in combination with the previous example. Thus, the pulsing of the heater illustrated in Fig. 12a, may also be performed in combination with the modulation of the optical pump beam. The pulsing of the heater may therefore be synchronised with the modulation of the intensity of the optical pump beam such that the heater applies heat to the optical cell when the optical pump has the relatively higher intensity.

As described in relation to the interventional device 110 above, in some examples the magnetic sensor 120 includes a plurality of magnetic sensor elements. Each magnetic sensor element is configured to detect magnetic fields intercepting a different volume of the interventional device 110. These examples were described with reference to Fig. 10. In one example of the system 200 described with reference to Fig. 11, the controller 140 may be further configured to:

- measure a signal-to-noise ratio of the signals generated by the magnetic sensor elements; and to
- generate the detection result using the signal(s) from the one or more of the magnetic sensor elements having the highest signal-to-noise ratio.

With reference to Fig. 10, in this example, an example of a magnetic sensor element is the magnetic sensor element that is provided by the uppermost optical path in the optical cell 130 that is closest to the bundle of nerve fibers BNFi, together with its associated input and output optical fibers IPFi, OPFi and lenses IPLi, OPLi. In this example arrangement, the activation of the bundle of nerve fibers BNFi is expected to generate a signal with a higher signal to noise ratio than the lowermost magnetic sensor element in Fig. 10. Thus, in this example, a detection result would be generated using the signal(s) from the uppermost magnetic sensor elements that has the highest signal-to-noise ratio. By using the sensor element, or elements with the highest signal to noise ratio to generate the detection result, the overall signal to noise ratio of the detection result may be improved. This in turn improves the accuracy of the detection result.

In this example, the signal-to-noise ratio of the signals generated by the magnetic sensor elements may be measured in various ways. For instance, a scanning procedure may be used wherein each of the individual signals from the separate magnetic sensor elements are measured sequentially. The scanning procedure may for example include measuring the signals generated by each of the magnetic sensor elements in a sequence that progresses around an axis of the interventional device. Alternatively, the individual signals from the separate magnetic sensor elements may all be measured simultaneously. By further determining, and outputting, a signal indicative of the magnetic sensor element having the signal(s) with the highest signal-to-noise ratio, a direction in which the activated nerve is located, may be determined. This information may be used by a physician to determine in which direction to steer the interventional device in order to perform a treatment on the activated nerve.

Thus, in one example, the sensor elements are distributed around an axis of the interventional device 110 such that the sensor elements generate signals in response to magnetic fields produced at different orientations around the axis; and the controller 140 is further configured to identify an orientation around the axis of the interventional device 110 at which the measured signals have a maximum signal-to-noise ratio.

In this example, the controller 140 may identify the orientation of the maximum signal-to-noise ratio in various ways. For example, the controller may output a graphical representation of the orientation with the maximum signal-to-noise ratio to a display device. The graphical representation may for instance include an icon representing the interventional device and the relative orientation in which the measured signals with the maximum signal-to-noise ratio have been measured.

In this example the interventional device 110 may also include one or more actuators for adjusting a position and/or orientation of the insertable portion of the interventional device. The one or more actuators may also be used with the single sensor example illustrated in Fig. 7. The one or more actuators are configured to automatically steer the insertable portion in a direction of the orientation having the maximum signal-to-noise ratio.

This example therefore provides an automatic re-positioning of the interventional device towards a bundle of nerve fibers that produced the magnetic fields. The actuator(s) may be provided by an expandable balloon, such as an angioplasty balloon for example, or alternatively by the actuators of a steerable catheter, or guidewire manipulator, for example. In the former case, one or more expandable balloons may be positioned around the axis of the interventional device 110 such that when expanded, the expandable balloon forces the magnetic sensor element on the opposing side of the axis transaxially in the direction of the orientation having the maximum signal-to-noise ratio. This example is described with reference to Fig. 13, which is a schematic diagram illustrating an example of a cross section of a vessel VES shrouded by bundles of nerve fibers $BNF_{1..3}$, and an interventional device ID including an expandable balloon, in accordance with some aspects of the present disclosure. As illustrated in Fig. 13, the interventional device includes a plurality of magnetic sensors $120_{1..4}$. In this example, the orientation having the maximum signal-to-noise ratio has been identified as the direction of the bundle of nerve fibers $BNF_2$. In response to this identification, the expandable balloon BAL has been inflated automatically in order to steer the insertable portion of the interventional device in the direction of the bundle of nerve fibers $BNF_2$. The interventional device ID may also include one or more additional expandable balloons (not illustrated in Fig. 13) for steering the interventional device ID in different directions. In one example, one or more expandable balloons that operate in a similar manner to the expandable balloon BAL are coupled to the interventional device in locations that are diametrically opposite a respective magnetic sensor $120_{1..4}$. Thus, when the expandable balloon BAL expands, the interventional device is moved within the lumen such that the magnetic sensor $120_{1..4}$ is moved closer to the vessel wall.

After the interventional device ID illustrated in Fig. 13 has been moved closer to the vessel wall by the expandable balloon(s), a map of the magnetic field around the circumference of the vessel may be generated by rotating the interventional device ID around the longitudinal axis of the interventional device. Similarly, a map of the magnetic field along the vessel may be generated by translating the interventional device ID along the axis of the vessel. The rotation and translation may be provided by a user in response to instructions provided by the controller 140. Alternatively, the controller may generate control signals for controlling a manipulator, or a robot, to provide the desired rotation/ translation. The balloon may be expanded such that the magnetic sensor $120_{1..4}$ contacts the vessel wall, for example. By using the expandable balloon(s) to move the magnetic sensor $120_{1..4}$ closer to the vessel wall in this manner, the separation between the magnetic sensor $120_{1..4}$ and the nerves that generate the magnetic fields is reduced, which results in an increase in the detected magnetic field strength. By expanding the balloon such that the magnetic sensor $120_{1..4}$ contacts the vessel wall, the reliability of the map(s) of the magnetic fields is improved because the separation between the magnetic sensor $120_{1..4}$ and the nerves that generate the magnetic fields is controlled.

A map of the magnetic field around the circumference of the vessel, or along the vessel, may be generated in a similar manner using an interventional device that includes a single magnetic sensor 120 and one or more expandable balloons. Thus, similar maps may be generated by attaching one or more expandable balloons to the interventional device illustrated in Fig. 7, and rotating or translating the interventional device as described above.

As mentioned above with reference to Fig. 6, the nerve activity that is sensed by the sensor 120 via the detected magnetic fields may be triggered artificially, or it may be triggered naturally, i.e. spontaneously. The inventors have determined that for spontaneously-activated nerves, the signature of the signals generated by the magnetic sensor 120, can be used as a measure of renal nerve sympathetic overdrive. As mentioned above, renal nerve sympathetic overdrive is an indicator for hypertension, i.e. elevated blood pressure.

Fig. 14 is a graph illustrating a) a signature of a pulse train representing the magnetic field detected from a renal nerve in a non-hypertensive subject and b) a signature of a pulse train representing the magnetic field detected from a renal nerve in a hypertensive subject in whom the renal nerve activity is increased, in accordance with some aspects of the present disclosure. In Fig. 14, normalized magnetic field strength is plotted on each of the vertical axes, against time in seconds, on each of the horizontal axes, for the non-hypertensive subject, and for the hypertensive subject. Significant differences may be observed between the upper graph for a non-hypertensive subject, and the lower graph for a hypertensive subject. These include differences in: a period of the pulses, the duration of a pulse train, and the number of pulses in a pulse train. These differences may therefore be used to distinguish between a non-hypertensive subject and a hypertensive subject.

In one example of the system 200 described with reference to Fig. 11, the controller 140 may be further configured to identify a signature in the received signals; and to output the detection result indicative of nerve activity in response to the received signals by outputting a measure of renal nerve sympathetic overdrive based on the identified signature.

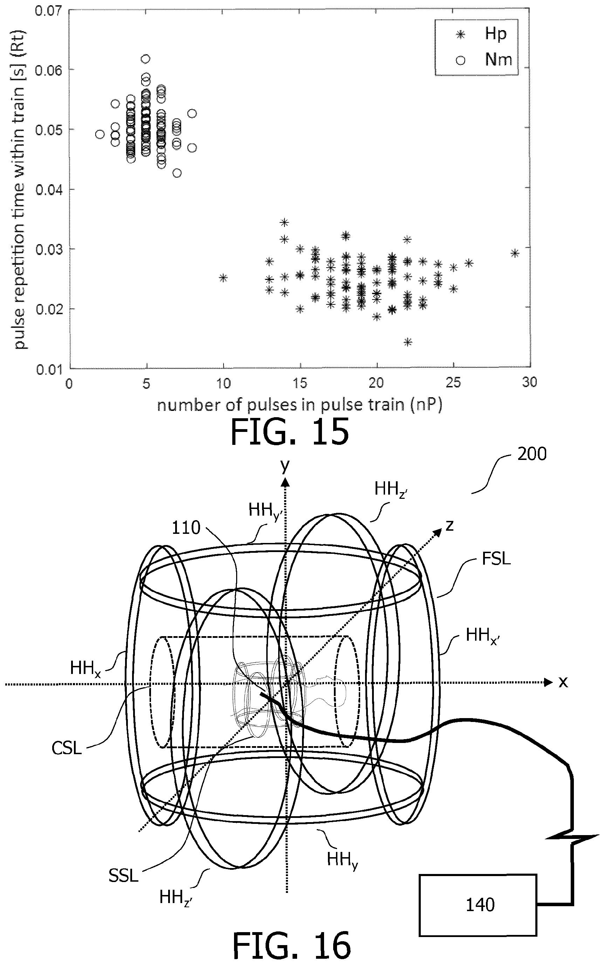

In this example, the signature of the received signals may for example include one or more of: a period of one or more pulses in a pulse train in the signals generated in response to magnetic fields produced by spontaneous nerve activity, a duration of a pulse train in the signals generated in response to magnetic fields produced by spontaneous nerve activity, and a count of the number of pulses in a pulse train in the signals generated in response to magnetic fields produced by spontaneous nerve activity. Also, the number of pulses per second, measured for instance over a period of one minute, differs significantly between the two groups. A clear distinction between these groups of subjects is illustrated in Fig. 15, which illustrates a plot of the pulse repetition time within a pulse train, Rt, against the number of pulses in a pulse train, nP, for simulated magnetic fields in a group of non-hypertensive subjects (circle symbols) and hypertensive subjects (stars), in accordance with some aspects of the present disclosure.

The inventors have also determined that the signals measured by the magnetic sensor 120 described above with reference to Fig. 6 and Fig. 11, are susceptible to interference from various sources of magnetic fields that are unrelated to nerve activity. In general, magnetic fields are present everywhere, including from the earth's magnetic field and electronic devices. Magnetic fields may also be generated within a subject by physiological signals that have a different origin that the nerve of interest. Magnetic fields may also be generated by physiological signals in other subjects that are in the vicinity of a subject of interest. The presence of large background magnetic fields presents a challenge to sensitive magnetic field sensors since these magnetic fields may saturate a magnetic sensor. In the examples described below, various implementations are described in which magnetic field compensation is used to compensate for such magnetic field interference.

In one example, active compensation of global magnetic fields is used to reduce the impact of magnetic field interference on the magnetic sensor 120. In this example, the interventional device 110, and also the subject in which the nerve activity is measured, are surrounded by a plurality of Helmholtz coils. For example, three pairs of Helmholtz coils may be used, wherein each pair is separated along one of the orthogonal axes x, y, and z. An example of this arrangement is illustrated in Fig. 16, which includes three pairs of Helmholtz coils $HH_{x,x'}$, $HH_{y,y'}$ and $HH_{z,z'}$, wherein each pair is separated along the corresponding orthogonal axes x, y and z. Active compensation of magnetic field interference is achieved by adjusting the currents in each of the coils in real-time in response to measurements of the magnetic field interference. The currents in the coils generate magnetic fields within the volume encompassed by the coils, which has the effect of reducing the magnetic field interference. In so doing, the Helmholtz coils can be used to compensate for both static magnetic field interference from e.g. the earth's magnetic field, as well as deterministic magnetic field interference that is caused by e.g. electronic equipment.

In another example, passive magnetic field shielding is used to reduce the impact of magnetic field interference on the magnetic sensor 120. In this example, the interventional device 110, and also the subject in which the nerve activity is measured, are surrounded by a magnetic shielding material. The interventional device 110 and the subject may for example be surrounded by a magnetic shielding material in the form of a cylinder. Effective magnetic shielding materials typically have a high value of magnetic permeability. One example of such materials is MuMetal, which is a Nickel-Iron alloy. Some Nickel-Iron MuMetals that provide a high degree of magnetic shielding have more than 40%, of Nickel.

In another example, active compensation of local magnetic fields is used to reduce the impact of magnetic field interference on the magnetic sensor 120. In this example, the interventional device 110, and also a portion of the subject in which the nerve activity is measured, are surrounded by a plurality of Helmholtz coils. This example is similar to the aforementioned example of active compensation for global magnetic fields that was described above, with the difference that only a portion of the subject, and not the entire subject, is surrounded by the plurality of Helmholtz coils. This example operates in the same manner as described above for the active compensation for global magnetic fields. In this example, the local magnetic fields that are compensated for may include magnetic fields that are generated within the subject, other than the nerve of interest. For example, this example may be used to reduce the impact of magnetic field interference from the beating heart of the subject.

All three of these examples of magnetic shielding may also be used together in order to improve the degree of shielding against magnetic field interference. An example of an arrangement in which these three forms of magnetic shielding are used together, is described with reference to Fig. 16, which illustrates an example of a system 200 that includes nested magnetic field shielding arrangement, in accordance with some aspects of the present disclosure. In this example, the system 200 includes a nested magnetic field shielding arrangement comprising a first, outermost, layer, of magnetic shielding FSL comprising a plurality of Helmholtz coils configured to provide active compensation for global magnetic fields generated outside a subject; and a second, innermost, layer of magnetic shielding SSL comprising a plurality of Helmholtz coils configured to provide active compensation for local magnetic fields generated within a subject, and an third layer of magnetic shielding CSL sandwiched between the first and second layers, the third layer of magnetic shielding comprising a passive magnetic shielding material.

In so doing, it may be provided that the output of the magnetic field sensor 120 remains sensitive to the weak magnetic fields generated by nerve activation, and also avoids saturation as a result of magnetic field interference.

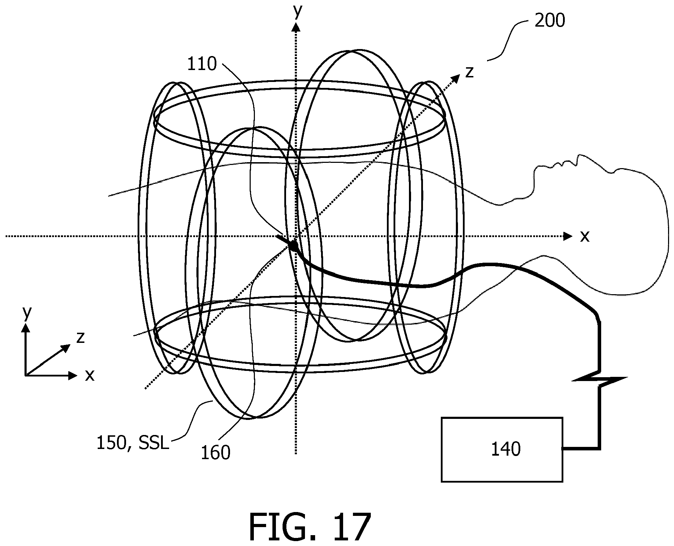

The inventors have also determined that despite the use of the aforementioned arrangement to compensate for magnetic field interference, there may be some residual magnetic field interference within a volume encompassed by the second, innermost, layer of magnetic shielding SSL. The origin of this interference may be external to the second, innermost, layer of magnetic shielding SSL, or alternatively the origin may be within the second, innermost, layer. For instance, this interference may originate from the earth's magnetic field, or it may be generated within a subject by another source than the nerve of interest. For instance, it may have its origin in the subject's beating heart. This interference may vary with position within the volume encompassed by the second, innermost, layer of magnetic shielding SSL. For instance, it may increase as the magnetic sensor 120 is moved toward the heart. In order to compensate for such spatial dependence of the interference, in one example, a system is provided in which the interventional device includes a motion sensor 160. This example is described with reference to Fig. 17, which illustrates an example of a system 200 that includes an interventional device 110, a motion sensor 160, and one or more coils 150, SSL that are configured to generate a magnetic field for compensating for a background magnetic field detected by the magnetic sensor 120, in accordance with some aspects of the present disclosure. In this example, the system 200 includes one or more coils 150, SSL that are configured to generate a magnetic field for compensating for, i.e. reducing a magnitude of, a background magnetic field detected by the magnetic sensor 120. The interventional device 110 further comprises a motion sensor 160. The motion sensor 160 is mechanically coupled to the interventional device 120 for detecting a motion of the magnetic sensor 120. The controller 140 is also configured to:

- measure the background magnetic field based on signals generated by the magnetic sensor 120 in the absence of magnetic fields produced by nerve activity;
- apply signals to the one or more coils 150, SSL in order to compensate for the background magnetic field during the measurement of magnetic fields produced by nerve activity; and
- repeat the measurement of the background magnetic field, and the corresponding applying of signals to the one or more coils, in response to a detection of a motion of the interventional device 110 by the motion sensor.

The effect of the operations performed by the controller in this example is to recalibrate the magnetic field compensation that is provided by the one or more coils in the event of movement of the interventional device. In so doing, as the interventional device is moved within the volume encompassed by the second, innermost, layer of magnetic shielding SSL, variations in magnetic field interference, due to e.g. changes in the proximity between the magnetic sensor and the beating heart, may be compensated-for. In this example, the one or more coils, 150, SSL may for instance be provided by the second, innermost, layer of magnetic shielding SSL that includes a plurality of Helmholtz coils, and which were described above with reference to Fig. 16. Alternatively, the one or more coils 150, SSL may form part of the interventional device. For example, the one or more coils 150, SSL may be coupled to a guide catheter, and through which guide catheter the interventional device 110 is inserted into a subject.

In this example, the motion sensor may be provided by various devices, including one or more of an accelerometer, a gyroscope, and an optical fiber-based position tracking system, for example. An example of an optical fiber-based position tracking system is described in a document WO 2013/136247 A1. This system determines a position and a shape of an object based on an amount of strain induced in one or more fiber Bragg gratings.

In a related example, the system described with reference to Fig. 17 provides compensation for magnetic field interference that is generated by cardiac activity in the subject in which nerve activity is measured using an electrocardiogram, ECG, signal. In this example, the signals that are generated by the magnetic sensor 120 are generated in response to magnetic fields produced by nerve activity in a subject, and the background magnetic field is generated at least in part by cardiac activity in the subject. In this example, the controller 140 is further configured to receive an electrocardiogram, ECG, signal for the subject; and the controller 140 is configured to apply signals to the one or more coils in order to compensate for the background magnetic field during the measurement of magnetic fields produced by nerve activity, by applying time-dependent signals to the one or more coils in synchronisation with the received ECG signal in order to compensate for the cardiac activity.

In this example, the electrocardiogram, ECG, signal for the subject may be generated using various devices, including using ECG electrodes that are applied to the subject. The heart may be considered to be the strongest common source of magnetic interference in the vicinity of the kidneys. The magnetic field generated by the heart has a peak during the depolarization of the cardiac ventricle. This magnetic field may exceed the dynamic range of some magnetic sensors, and therefore risks saturating the output of such sensors. The implementation described in this example reduces the risk of saturation by reducing the impact of the heart's magnetic field on the magnetic sensor. In this example, the compensating magnetic field may be applied in synchronisation with a position on the received ECG signal, such as the R peak of the ECG signal, for example.

In another related example, the system described with reference to Fig. 17 provides compensation for magnetic field interference using a 3D background magnetic field map. In this example, the controller 140 is further configured to:

- receive tracking data representing a position of the magnetic sensor 120 within a sensing region;
- receive a 3D background magnetic field map representing a background magnetic field distribution in the sensing region; and

- estimate a magnitude of the background magnetic field distribution at the position of the magnetic sensor 120 within the sensing region based on the received tracking data and the received 3D background magnetic field map; and

- the controller 140 is configured to apply signals to the one or more coils in order to compensate for the background magnetic field during the measurement of magnetic fields produced by nerve activity, based at least in part on the estimated magnitude of the background magnetic field distribution at the position of the magnetic sensor 120.

In this example, the tracking data may be provided by various tracking systems, including the aforementioned optical fiber-based position tracking system described in a document WO 2013/136247 A1, or by tracking the position of the magnetic sensor 120 using an imaging system. The imaging system may be a projection X-ray imaging system, or a CT imaging system, or an ultrasound imaging system, for example. For instance, a shape of the magnetic sensor, or a shape of a fiducial marker identifying its location, may be tracked in image data generated by such imaging systems in order to determine the position of the magnetic sensor. The 3D background magnetic field map may be measured using the magnetic sensor 120 by positioning the magnetic sensor at multiple positions within the volume in which magnetic fields are to be compensated for by the one or more coils 150, SSL in the absence of a patient in the volume. After positioning the subject in this volume, the position of the magnetic sensor 120, as determined by the tracking data, is used to estimate the value of the background magnetic field at the current position of the magnetic sensor by interrogating the 3D background magnetic field map. The controller 140 then applies signals to the one or more coils in order to compensate for the background magnetic field, by providing a field that compensates for the estimated the value of the background magnetic field at the current position of the magnetic sensor, as determined by interrogating the 3D background magnetic field map.

The operations described above that are performed by the controller 140 may be provided in the form of a computer implemented method for use with the system 200 described above. Thus, in one example, a computer implemented method for use with the system 200 includes instructions to:

- receive the signals generated by the magnetic sensor 120, and to output a detection result indicative of nerve activity in response to the received signals.

Other operations described above as being performed by the controller 140 may similarly be provided as instructions of the computer implemented method.

The operations described above that are performed by the controller 140 may be provided in the form of a computer program product for use with the system 200 described above. Thus, in one example, a computer program product for use with the system 200 comprises instructions which when executed by one or more processors, cause the one or more processors to:

- receive the signals generated by the magnetic sensor 120, and to output a detection result indicative of nerve activity in response to the received signals.

Other operations described above as being performed by the controller 140 may similarly be provided as instructions of the computer program product.

The above examples are to be understood as illustrative of the present disclosure, and not restrictive. Further examples are also contemplated. For instance, examples described in relation to the interventional device 110 may also be included within the system 200. Moreover, it is noted that examples described in relation to the system 200, include operations that are performed by a controller. These operations may be provided in the form of instructions on a computer program product, and which when executed by one or more processors, cause the one or more processors to carry out the instructions. Similarly, these operations may be provided in the form of instructions stored on a non-transitory computer-readable storage medium, and which, when executed by at least one processor, cause the at least one processor to carry out the instructions. It is to be understood that a feature described in relation to any one example may be used alone, or in combination with other described features, and may be used in combination with one or more features of another of the examples, or a combination of other examples. Furthermore, equivalents and modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

**Claims**

1. An interventional device (110) for detecting nerve activity, the interventional device comprising:

 a magnetic sensor (120);
 wherein the magnetic sensor (120) is coupled to an insertable portion of the interventional device;
 and wherein the magnetic sensor (120) is configured to generate signals in response to magnetic fields produced by nerve activity.

2. The interventional device (110) according to claim 1, wherein the magnetic sensor (120) comprises an optically pumped magnetometer, OPM;

   wherein the OPM comprises an optical cell (130) containing an alkali metal in a liquid and/or a gaseous phase; and
   wherein the OPM is configured to generate the signals in response to magnetic fields produced by nerve activity by measuring a magnetic-field-dependent optical property of the alkali metal.

3. The interventional device (110) according to claim 2, wherein the optical cell (130) further contains a background gas; and
   wherein the OPM is further configured to generate the signals in response to magnetic fields produced by nerve activity by measuring a magnetic-field-dependent optical property of the background gas.

4. The interventional device (110) according to claim 2 or claim 3, wherein the OPM further comprises a heater;
   wherein the heater is configured to supply heat to the optical cell (130).

5. A system (200) comprising the interventional device (110) according to any of the previous claims, and a controller (140);
   wherein the controller is configured to receive the signals generated by the magnetic sensor (120), and to output a detection result indicative of nerve activity in response to the received signals.

6. The system (200) according to claim 5 when dependent on claim 4, wherein the controller (140) is further configured to selectively operate the OPM in a relatively lower sensitivity mode and in which a relatively lower power level is supplied by the heater to the optical cell (130), and in a relatively higher sensitivity mode and in which a relatively higher power level is supplied by the heater to the optical cell (130), in order to generate the signals in response to magnetic fields produced by nerve activity.

7. The system (200) according to claim 6, wherein:

   the optical cell (130) contains an alkali metal in a liquid and/or a gaseous phase, and a background gas; and the OPM is configured to generate the signals in response to magnetic fields produced by nerve activity by measuring a magnetic-field-dependent optical property of the alkali metal and the background gas, and wherein in the relatively lower sensitivity mode an optical property of the background gas is sensed, and wherein in the relatively higher sensitivity mode an optical property of the alkali metal is sensed; or
   the optical cell (130) contains a first alkali metal in a liquid and/or a gaseous phase, and a second alkali metal in a liquid and/or a gaseous phase; and the OPM is configured to generate the signals in response to magnetic fields produced by nerve activity by measuring a magnetic-field-dependent optical property of the first alkali metal and the second alkali metal, and wherein in the relatively lower sensitivity mode an optical property of the first alkali metal is sensed, and wherein in the relatively higher sensitivity mode an optical property of the second alkali metal is sensed.

8. The system (200) according to claim 5 when dependent on claim 4, wherein the interventional device further comprises a temperature sensor;

   wherein the temperature sensor is in thermal contact with i) an outer surface of the interventional device, or ii) the optical cell (130); and
   wherein the controller (140) is configured to operate the heater in response to a temperature measured by the temperature sensor.

9. The system (200) according to claim 5 when dependent on claim 2, wherein the OPM comprises an optical source configured to provide an optical pump beam, and wherein the OPM is configured to measure the magnetic field-dependent optical property of the alkali metal in response to an excitation of the alkali metal within the optical cell (130) by the optical pump beam;

   wherein the controller (140) is configured to modulate an intensity of the optical pump beam between a relatively lower intensity and a relatively higher intensity; and
   wherein the OPM is configured to measure the magnetic field-dependent optical property of the alkali metal by determining a transmission of the optical pump beam through the optical cell (130) whilst the optical pump beam excites the alkali metal at the relative higher intensity.

10. The system (200) according to claim 5 wherein the magnetic sensor (120) comprises a plurality of magnetic sensor elements; and

    wherein each magnetic sensor element is configured to detect magnetic fields intercepting a different volume of the interventional device (110); and
    wherein the controller (140) is further configured to:

measure a signal-to-noise ratio of the signals generated by the magnetic sensor elements; and to

generate the detection result using the signal(s) from the one or more of the magnetic sensor elements having the highest signal-to-noise ratio.

11. The system (200) according to claim 10, wherein the sensor elements are distributed around an axis of the interventional device (110) such that the sensor elements generate signals in response to magnetic fields produced at different orientations around the axis; and;

wherein the controller (140) is further configured to identify an orientation around the axis of the interventional device (110) at which the measured signals have a maximum signal-to-noise ratio.

12. The system (200) according to claim 5, wherein the controller (140) is further configured to identify a signature in the received signals; and

to output the detection result indicative of nerve activity in response to the received signals by outputting a measure of renal nerve sympathetic overdrive based on the identified signature.

13. The system (200) according to claim 5, further comprising one or more coils (150, SSL) configured to generate a magnetic field for compensating for a background magnetic field detected by the magnetic sensor (120); and

wherein the interventional device (110) further comprises a motion sensor (160);
wherein the motion sensor (160) is mechanically coupled to the interventional device (120) for detecting a motion of the magnetic sensor (120); and
wherein the controller (140) is further configured to:

measure the background magnetic field based on signals generated by the magnetic sensor (120) in the absence of magnetic fields produced by nerve activity;
apply signals to the one or more coils (150, SSL) in order to compensate for the background magnetic field during the measurement of magnetic fields produced by nerve activity; and
repeat the measurement of the background magnetic field, and the corresponding applying of signals to the one or more coils, in response to a detection of a motion of the interventional device (110) by the motion sensor.

14. The system (200) according to claim 13, wherein the signals are generated in response to magnetic fields produced by nerve activity in a subject, and wherein the background magnetic field is generated at least in part by cardiac activity in the subject; and

wherein the controller (140) is further configured to receive an electrocardiogram, ECG, signal for the subject; and
wherein the controller (140) is configured to apply signals to the one or more coils in order to compensate for the background magnetic field during the measurement of magnetic fields produced by nerve activity, by applying time-dependent signals to the one or more coils in synchronisation with the received ECG signal in order to compensate for the cardiac activity.

15. The system (200) according to claim 13 or claim 14, wherein the controller (140) is further configured to:

receive tracking data representing a position of the magnetic sensor (120) within a sensing region;
receive a 3D background magnetic field map representing a background magnetic field distribution in the sensing region; and
estimate a magnitude of the background magnetic field distribution at the position of the magnetic sensor (120) within the sensing region based on the received tracking data and the received 3D background magnetic field map; and
wherein the controller (140) is configured to apply signals to the one or more coils in order to compensate for the background magnetic field during the measurement of magnetic fields produced by nerve activity, based at least in part on the estimated magnitude of the background magnetic field distribution at the position of the magnetic sensor (120).

FIG. 1

FIG. 2

a)

b)

FIG. 3

Magnetic Flux Density:
Magnitude (T)

a)

6.12e-11

4.59e-11

3.06e-11

1.53e-11

0.000

b)

FIG. 4

FIG. 5

FIG. 6

FIG. 7

FIG. 8

FIG. 9

FIG. 10

FIG. 11

a)

$P_{HEATER}$

Time

$t_0$  $t_1$  $t_2$  $t_3$  $t_4$  $t_5$

b)

$T_{Th}$

$T_{CELL}$

$T_T$

Time

c)

$B_{NERVE}$

Time

d)

$B_{DETECTED}$

Time

## FIG. 12

$120_4$

VES

$BNF_1$

BAL

ID

$120_1$

$BNF_2$

$BNF_3$

$120_2$

$120_3$

## FIG. 13

FIG. 14

EP 4 285 821 A1

FIG. 15

FIG. 16

FIG. 17

Europäisches
Patentamt

European
Patent Office

Office européen
des brevets

# EUROPEAN SEARCH REPORT

**Application Number**

EP 22 17 2894

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2013/096461 A1 (CARDIAC PACEMAKERS INC [US]) 27 June 2013 (2013-06-27) | 1-5,8-12 | INV.<br>A61B5/40<br>A61B5/311<br>A61B5/388<br>A61B5/242 |
| Y | * the whole document * | 13 | |
| X | US 2019/223777 A1 (DUBHASHI ABHIJEET [US] ET AL) 25 July 2019 (2019-07-25)<br>* paragraphs [0033] – [0035] *<br>* paragraph [0039] * | 1,2,4,5,9,10,12 | |
| | | | ADD.<br>A61B5/05<br>A61B5/20 |
| X | US 2010/219820 A1 (SKIDMORE FRANK M [US] ET AL) 2 September 2010 (2010-09-02)<br>* paragraphs [0001] – [0015] *<br>* paragraphs [0120] – [0123] * | 1,2,4,5,10 | |
| Y | US 2021/244329 A1 (LEDBETTER MICAH [US] ET AL) 12 August 2021 (2021-08-12)<br>* paragraphs [0013] – [0025] *<br>* paragraphs [0079] – [0090] *<br>* paragraphs [0100], [0126], [0142], [0173] *<br>* paragraphs [0166] – [0167] * | 13 | |
| | | | **TECHNICAL FIELDS SEARCHED (IPC)** |
| A | US 2021/251542 A1 (JOHNSON BRYAN [US] ET AL) 19 August 2021 (2021-08-19)<br>* paragraphs [0072] – [0076] * | 1-15 | A61B |
| E | WO 2022/182526 A1 (HI LLC [US]) 1 September 2022 (2022-09-01)<br>* paragraphs [0080] – [0081] * | 1-15 | |

The present search report has been drawn up for all claims

| Place of search | Date of completion of the search | Examiner |
|---|---|---|
| The Hague | 6 October 2022 | Loveniers, Kris |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
........................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 17 2894

This annex lists the patent family members relating to the patent documents cited in the above-mentioned European search report.
The members are as contained in the European Patent Office EDP file on
The European Patent Office is in no way liable for these particulars which are merely given for the purpose of information.

06-10-2022

| Patent document cited in search report | | Publication date | Patent family member(s) | | Publication date |
|---|---|---|---|---|---|
| WO 2013096461 | A1 | 27-06-2013 | EP | 2793723 A1 | 29-10-2014 |
| | | | US | 2014073903 A1 | 13-03-2014 |
| | | | WO | 2013096461 A1 | 27-06-2013 |
| US 2019223777 | A1 | 25-07-2019 | US | 2019223777 A1 | 25-07-2019 |
| | | | US | 2022133210 A1 | 05-05-2022 |
| | | | WO | 2019147472 A1 | 01-08-2019 |
| US 2010219820 | A1 | 02-09-2010 | US | 2009149736 A1 | 11-06-2009 |
| | | | US | 2010219820 A1 | 02-09-2010 |
| | | | WO | 2008127720 A2 | 23-10-2008 |
| US 2021244329 | A1 | 12-08-2021 | NONE | | |
| US 2021251542 | A1 | 19-08-2021 | US | 2020196932 A1 | 25-06-2020 |
| | | | US | 2021251542 A1 | 19-08-2021 |
| | | | WO | 2020131148 A1 | 25-06-2020 |
| WO 2022182526 | A1 | 01-09-2022 | US | 2022274010 A1 | 01-09-2022 |
| | | | WO | 2022182526 A1 | 01-09-2022 |

EPO FORM P0459

For more details about this annex : see Official Journal of the European Patent Office, No. 12/82

**EP 4 285 821 A1**

**Patent documents cited in the description**

- WO 2013096461 A1

- WO 2013136247 A1